(19) Europäisches Patentamt
European Patent Office
Office européen des brevets

(11) Publication number: **0 019 388**
**B1**

(12) **EUROPEAN PATENT SPECIFICATION**

(45) Date of publication of patent specification: **23.03.83**

(51) Int. Cl.[3]: **C 07 C 37/02,**
C 07 C 37/64,
C 07 C 39/24,
C 07 C 39/44,
C 07 C 79/46,
C 07 C 103/76,
C 07 C 121/75

(21) Application number: **80301401.8**

(22) Date of filing: **29.04.80**

(54) **Preparation of trifluoromethyl-substituted phenols and phenates and the preparation, from these phenols and phenates, of nitro- and trifluoromethyl-substituted diphenyl ethers.**

(30) Priority: **02.05.79 US 71341**

(43) Date of publication of application:
**26.11.80 Bulletin 80/24**

(45) Publication of the grant of the patent:
**23.03.83 Bulletin 83/12**

(84) Designated Contracting States:
**BE CH DE FR GB IT LI NL**

(56) References cited:

**DE - A - 2 753 900**
**GB - A - 1 110 232**

**Chemical Abstracts vol. 74, no. 11, 15 March 1971 Columbus, Ohio, USA J. S. BRADSHAW et al. "Reaction of monohalonaphthalenes with potassium tertbutoxide and tert-butyl alcohol in dimethyl sulfoxide" column 2, page 320, abstract no. 53314q**

(73) Proprietor: **Rohm and Haas Company**
**Independence Mall West**
**Philadelphia, Pennsylvania 19105 (US)**

(72) Inventor: **Johnson, Wayne Orrin**
**346 Centennial Road**
**Warminster, Pennsylvania, 18974 (US)**

(74) Representative: **Wood, Peter Worsley Spencer et al,**
**Rohm and Haas Company Patent Department**
**Chesterfield House Barter Street**
**London, WC1A 2TP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Preparation of trifluoromethyl-substituted phenols and phenates and the preparation, from these phenols and phenates, of nitro- and trifluoromethyl-substituted diphenyl ethers

This invention concerns a process for preparing trifluoromethyl-substituted phenols and phenates, these phenols and phenates being key intermediates, *inter alia* in the preparation of optionally ring-substituted trifluoromethylphenyl nitrophenylether herbicides such as are, for example, disclosed in U.S. Patent Nos. 3,798,276; 3,928,416; 4,031,131; 4,063,929; 3,784,635; 4,087,272; 4,002,662; 4,001,005; 3,983,168; 3,979,437; 3,941,830; 3,907,866; 3,873,302; 3,954,829; 3,839,444; 3,957,865; 4,017,300 and 3,862,209.

There are various known routes to the intermediates which are prepared by the process of the invention and for such known routes reference is directed to, for example, U.S. Patent Nos. 3,888,932; 3,772,344; 3,819,755 and the references cited therein. Reference is also directed to U.K. Patent Specification No. 1,110,232, page 1, lines 57—72. This U.K. specification discloses that alkali or alkaline earth metal salts of perfluoroalkyl-substituted phenols may be prepared by treating a perfluoroalkyl (including trifluoromethyl)-substituted benzene containing at least one fluoro ring substituent with an alkali or alkaline earth metal hydroxide in the presence of an inert organic liquid solvent such as tertiary butanol or pyridine. Known processes either involve too many steps or are economically unattractive or both. [See Mooradian, et al., *J. Am. Chem. Soc., 73,* 3470—2 (1951); McBee et al., *J. Am. Chem. Soc. 73* 1325—6 (1951); Jones, R.G., *J. Am. Chem. Soc., 69,* 2346—50 (1947); and Lavagnino, E.R., et al., *Org. Preparations and Procedures International,* Vol. 9 pages 96—98 (1977)].

The basic problem in the preparation of a trifluoromethyl substituted phenol or phenate is the sensitivity of the trifluoromethyl radical to not only concentrated but even dilute base. (See Jones, R.G., *J. American Chemical Society, 69,* 2346—50 (1947).

It has now been discovered that by employing a special solvent system trifluoromethyl-substituted phenates can be produced in a single step and in good yields. The corresponding trifluoromethyl-substituted phenols can then be prepared by acidification.

In accordance with the invention, there is provided a process for preparing a substituted phenate or the corresponding phenol, said phenate being of the formula:

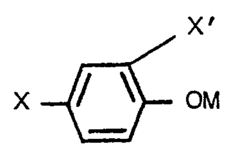

(I)

wherein X is halo or trifluoromethyl with the proviso that, when X is halo, X′ is trifluoromethyl; X′ is hydrogen, halo, trifluoromethyl or ($C_1$—$C_4$)alkyl; and M is the cation of an alkali metal or alkaline earth metal, the process comprising reacting a compound of the formula:

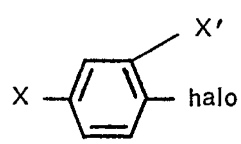

(wherein X and X′ are as above defined) with an alkali metal or alkaline earth metal hydroxide base in a solvent system which is substantially inert to said base characterized in that the solvent system employed comprises a polar, aprotic solvent with a dielectric constant in the range of from 30 to 70 and a non-nucleophilic hydroxy-containing solvent which increases the solubility of said base in the solvent system whereby a phenate is obtained and, if a phenol is the desired product, reacting the phenate with an acid to form the phenol. The term halo used in this specification means fluoro, chloro, bromo or iodo and preferred halo substituents are fluoro and chloro. When X′ is $C_1$—$C_4$ alkyl, it may be methyl, ethyl, *n*-propyl, *iso*-propyl, *n*-butyl, *sec*-butyl or *tert*-butyl.

When, as disclosed in U.K. Patent Specification No. 1,110,232 cited above, an inert organic solvent such as tertiary butanol is used as the sole solvent, the $CF_3$ group(s) in the starting material used in the process of the invention is or are affected to an adverse extent. Surprisingly the use of an additional aprotic solvent as required in the process of the invention renders the $CF_3$ group(s) less sensitive to the base.

The process of the invention, in contrast to the prior art, permits the use of an alkali metal or alkaline earth metal hydroxide base which is substantially free of water or which may be associated in

the solvent system with water; water, if present at all, is preferably present in amounts (based on the weight of base plus water) not exceeding 25% and more usually not exceeding 15%.

In the process of the invention there may be used hydroxides such as sodium hydroxide, potassium hydroxide, calcium hydroxide or magnesium hydroxide; sodium and potassium hydroxides are preferred, particularly the latter.

The process to form phenates is generally conducted at a temperature in the range of from about 50 to about 100°C and, preferably, in the range of from about 65 to about 85°C. The reaction time may vary widely as from about 5 hours to about 5.5 days, more usually from 10 to 55 hours. The reaction is generally conducted at atmospheric pressure.

Examples of the preferred polar, aprotic solvents used include dimethyl sulfoxide, tetrahydrothiophene-1,1-dioxide and hexamethylphosphoric triamide.

The non-nucleophilic, hydroxylated cosolvent which provides for increased solubility of the metal hydroxide is believed to impart stability by hydrogen bonding with the formed 4-trifluoromethylphenolate salt.

Non-sterically hindered alcohols such as methanol, ethanol, *n*-propanol and *iso*-propanol, however, may enter into competitive reactions with the 4-trifluoromethylphenolate resulting in formation of undesired by-products. Less nucleophilic *n*-alcohols ($C_4$ and above) are more desirable as cosolvents than the $C_1$—$C_3$ alcohols listed above.

The hydroxylated cosolvents employed in this invention include those having the following structural formula:

a)

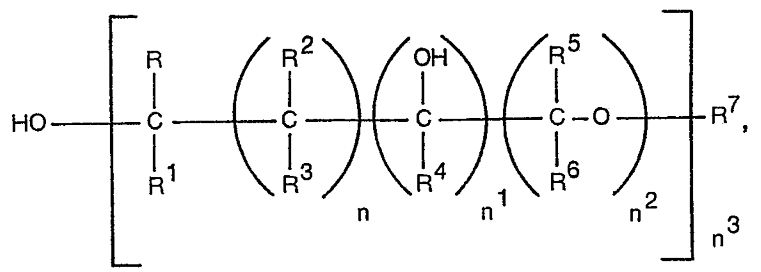

b)

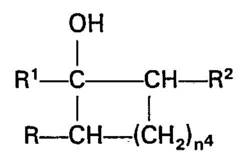

c) $C(CH_2OH)_4$

d)

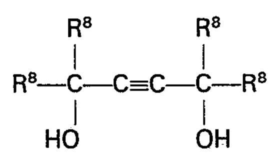

wherein R, $R^1$, $R^2$, $R^3$, $R^4$, $R^5$, $R^6$ and $R^7$ are the same or different groups selected from hydrogen; straight or branched lower alkyl (preferably ($C_1$—$C_4$)alkyl such as methyl); unsubstituted or substituted phenyl wherein the phenyl substituent(s) may be selected from lower alkyl (preferably ($C_1$—$C_4$)alkyl such as methyl), halo, lower alkoxy (preferably ($C_1$—$C_4$)alkoxy) and benzyl; $R^8$ is lower alkyl (preferably ($C_1$—$C_4$)alkyl such as methyl; n and $n^2$ are 0 or 1; $n^1$ is 0 to 2; $n^3$ is an integer of from 1 to 10 and $n^4$ is 2 to 4; provided that (a) $n^1$ is 2 only when n and $n^2$ are both 0, otherwise the sum of $n^1$ and $n^2$ must not be greater than 1, (b) (when $n^3$ is greater than 2) the sum of n and $n^2$ must not be greater than 1 and $n^1$ must be 0 and (c) (when $R^7$ is hydrogen) $n^1$ must be zero, the sum of n and $n^2$ must be 1 and at least 3 of R, $R^1$ plus either the pair $R^2$ and $R^3$ or the pair $R^5$ and $R^6$ must be other than hydrogen.

Examples of the hydroxylated solvents include *tert*-butyl alcohol, pinacol, pentaerythritol, 2,4-dimethyl-2,4-pentanediol, 2,4-dimethyl-2,3,4-pentanetriol, 2,4-dimethyl-1,2-pentanediol, 2,4-dimethyl-3-pentanol, 2,2,4-trimethyl-3-pentanol, 2,2-dimethyl-1-propanol,

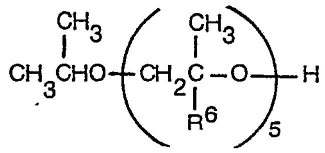 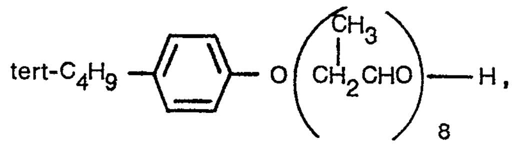

1-methyl-1-cyclohexanol, 2-*iso*-propyl-1-cyclopentanol and the like.

The cosolvents are usually employed at a ratio in the range of from about 1 to about 30 parts of the non-nucleophilic hydroxylated cosolvent to about 100 parts of the polar, aprotic solvent and preferably at a ratio in the range of from about 5 to about 20 parts of the non-nucleophilic solvent to about 100 parts of the polar, aprotic solvent, the above parts being by volume.

The following examples illustrate the process of this invention.

## Example 1

*2-Chloro-4-trifluoromethylphenol and potassium salt*

To a flask equipped with a stirrer, thermometer, condenser and drying tube was added 3,4-dichlorobenzotrifluoride (21.5 g, 0.10 mole), potassium hydroxide pellets (85% by weight KOH, balance water ≡ 0.20 mole KOH) and 100 ml of dimethyl sulfoxide (DMSO). The reaction mixture was warmed to 65°C for five hours during which time little reaction occurred. *Tert*-butyl alcohol (10 ml) was then added and the heating continued at 65°C over the weekend. The reaction mixture was cooled, poured slowly onto ice (400 g), and extracted with toluene (100 ml, then 25 ml) to remove any unreacted starting material. To the yellow aqueous layer containing 2-chloro-4-trifluoromethylphenol, potassium salt (still cold) there was added carbon tetrachloride (100 ml). The mixture was then acidified rapidly (to pH 1) with concentrated hydrochloric acid. The carbon tetrachloride layer was decanted, dried over anhydrous sodium sulfate and reduced under vacuum to afford 13.5 g of 2-chloro-4-trifluoromethylphenol. Distillation at 3.0 mm, afforded 10.8 g (69% yield) of substantially pure material, bp 79.5—80.5°C. Elemental Anal. Calcd. for $C_7H_4ClF_3O$: C, 42.75; H, 2.06; Cl, 18.04; F, 29.00; Found: C, 43.01; H, 2.24; Cl, 17.28; F, 28.52.

## Example 2

*Potassium salt of 2-chloro-4-trifluoromethylphenol*

To a flask equipped with a stirrer, thermometer, condenser and drying tube there was added 100 ml of dimethyl sulfoxide (DMSO), powdered potassium hydroxide pellets (85% by weight KOH, balance water ≡ 0.38 mole KOH), 3,4-dichlorobenzotrifluoride (21.5 g, 0.10 mole) and pinacol (10 g). The reaction mixture was warmed at 75°C for 52 hours, cooled and an aliquot analyzed by GLC to be 95% complete compared to unreacted 3,4-dichlorobenzotrifluoride. The desired potassium salt was isolated.

Variations of Example 1 used in the preparation of the potassium salt of 2-chloro-4-trifluoromethylphenol are illustrated in Table I below, Example 7 being included for comparison purposes to show the effect on yield of omitting the *tert*-butanol cosolvent.

TABLE I

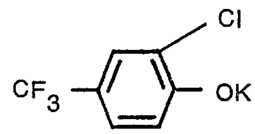

| Ex No. | Moles of Starting Compound* | Moles of KOH | Non-Nucleophilic Cosolvent (ml) | Polar, Aprotic Solvent (ml) | Time (hrs) | Temp. °C | Yield (%) |
|---|---|---|---|---|---|---|---|
| 3 | 0.1 | 0.2 | *tert*-butanol (10) | DMSO (100) | 65 | 65 | 69 |
| 4 | 0.1 | 0.48 | *tert*-butanol (20) | DMSO (100) | 132 | 65—75 | 73 |
| 5 | 0.1 | 0.45 | *tert*-butanol (20) | DMSO (100) | 47 | 62—75 | 90 |
| 6 | 2.5 | 8.76 | *tert*-butanol (500) | DMSO (2500) | 34 | 69—74 | 80 |
| 7 | 0.1 | 0.2 | — | DMSO (100 ml) | 5 | 65 | 0 |

* = 3,4-dichlorobenzotrifluoride

2-Trifluoromethylphenols can be prepared following the general procedures described in Examples 1 and 2, but employing 2-chloro- or 2-fluoro-benzotrifluoride; an example of such preparation follows.

Example 8

*4-Chloro-2-trifluoromethylphenol*

To a 300 ml, 3-necked flask fitted with a stirrer, condenser, thermometer and drying tube was added dimethylsulfoxide (100 ml), *tert*-butanol (20 ml), powdered potassium hydroxide pellets (30 g of 85% by weight KOH, balance water) and 2,5-dichlorobenzotrifluoride (21.5 g, 0.10 mole). The reaction mixture was warmed to 71—73°C for 60 hours; then additional KOH (10 g) was added and heating was continued at 73—75°C for 24 hours. The reaction mixture was cooled and the solvent partially removed by distillation at 0.9 mm, bp 55°C. The cooled pot residue was then poured into ice water (1000 g) which had been preacidified to pH 1 with concentrated HCl (50 ml). The aqueous mixture was extracted with carbon tetrachloride ($CCl_4$), the $CCl_4$ layer decanted and dried and the solvent removed under vacuum to afford 10.8 g of 4-chloro-2-trifluoromethylphenol, which was sublimed to afford pure product, mp 81—81.5°C. Elemental Anal. Calcd. for $C_7H_4ClF_3O$: C, 42.75; H, 2.06; Cl, 18.04. Found: C, 42.54; H, 2.36; Cl, 18.16.

The phenates of Formula I, and the corresponding phenols are of particular utility as intermediates in the preparation of herbicidal ethers of the formula:

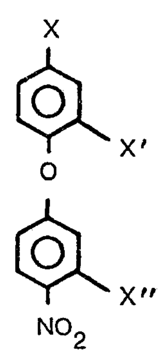

(II)

wherein X and X′ are as defined in Formula I and X″ is selected from hydrogen; halo; cyano; ($C_1$—$C_5$)alkyl (eg methyl, ethyl, *n*-propyl, *n*-butyl, *sec*-butyl, *tert*-butyl or *n*-pentyl); ($C_1$—$C_5$)alkoxy (eg methoxy, ethoxy, *iso*-propoxy, *n*-butoxy or *n*-pentoxy); carboxy in free acid or salt form; alkoxycarbonyl having 1 to 5 carbon atoms in the alkoxy moiety (eg methoxy, carbonyl, ethoxycarbonyl, *iso*-propoxycarbonyl, *tert*-butoxycarbonyl or *n*-pentoxycarbonyl); alkoxycarbonylalkoxy having 1 to 5 carbon atoms in each alkoxy moiety (eg 1-ethoxycarbonylethoxy or 1-methoxycarbonyl-*n*-propoxy); carbamoyl; mono- and di-($C_1$—$C_5$)alkyl substituted carbomoyl (eg N-methylcarbamoyl, N-*iso*-propylcarbamoyl, N-*n*-butylcarbamoyl, N,N-dimethylcarbamoyl or N,N-diethylcarbamoyl); substituted alkoxycarbonyl having 1 to 5 carbon atoms in said alkoxy moiety (eg 2-methoxyethoxycarbonyl, 2-ethoxy-*n*-propoxycarbonyl or 2-*iso*-butoxyethylcarbonyl); alkenyloxycarbonyl having 1 to 5 carbon atoms in the alkenyloxy moiety (eg 2-propenyloxycarbonyl or 2-(2-methylpropenyloxy)carbonyl); or trifluoromethyl.

One procedure for obtaining ethers of Formula II is illustrated by the following:

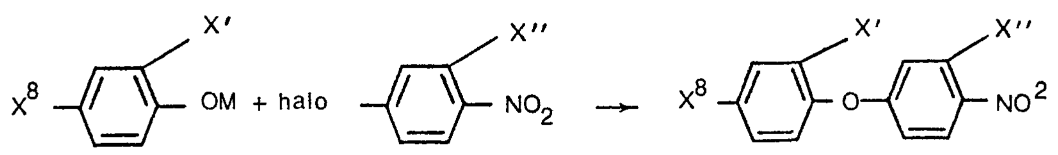

(I) (III) (II)

wherein M, X, X′ and X″ are as defined above and halo is preferably chloro or fluoro. Fluoro is preferred for enhancing reaction rates while chloro is preferred in terms of lower raw material costs. Instead of using as starting material a phenate of Formula I there may also be used the corresponding free phenol in conjunction with an alkali metal or alkaline earth metal base. This reaction to produce diphenyl ethers is preferably conducted in a polar aprotic solvent as described earlier using a temperature in the range of 20—130°C, there being optionally present a hydroxylated cosolvent as described earlier. It is therefore possible to use the same solvent system as is used for the preparation of the phenates.

The following illustrates in greater detail the preparation of the phenates, phenols and the preferred diphenyl ether herbicides. However, it is to be understood that other ethers falling within Formula II may also be prepared in a similar manner:

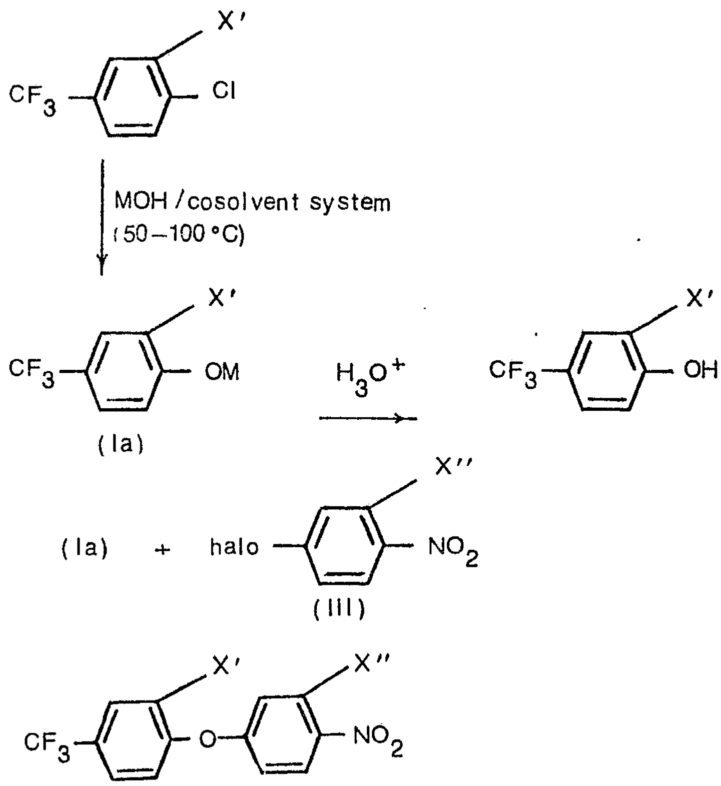


(Ia)

(Ia) +

(III)

(IIa)

OR

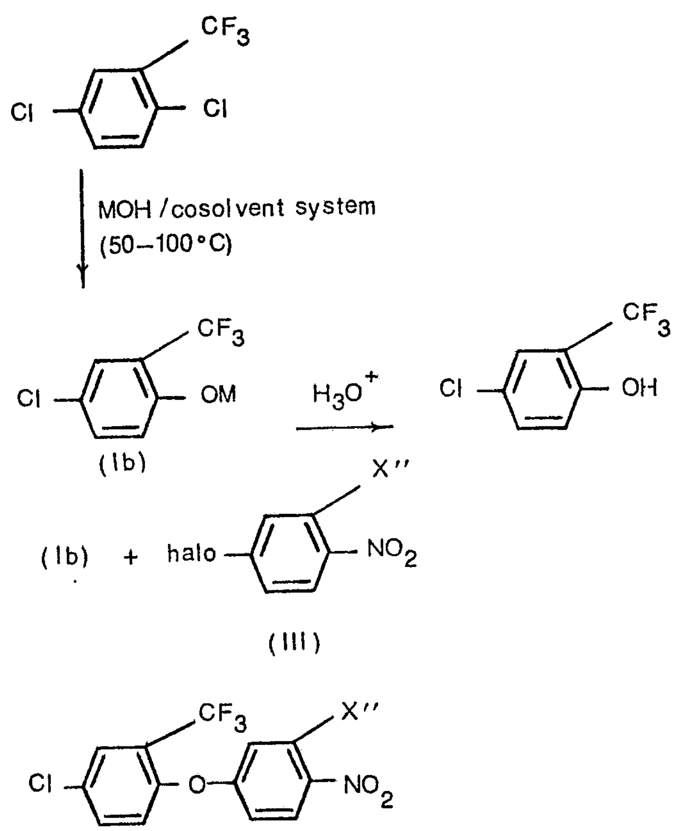


(Ib)

(Ib) +

(III)

(IIb)

wherein X′, X″ and M are as defined above. Especially preferred are those compounds where X′ is hydrogen or chloro and X″ is alkyl, alkenyl or alkoxyalkyl esters, or alkyl or dialkyl amides. The alkyl esters can be readily hydrolyzed under acidic or basic conditions to afford the carboxylic acid which can then be converted to alkali or alkaline earth metal salts or to various amine salts as desired.

The reaction between the trifluoromethylphenate of Formula I and the halonitrobenzene of Formula III is generally conducted at a temperature in the range of from about 25 to 130°C and preferably in the range of from about 25—85°C when "halo" is fluoro and 60—130°C, preferably 65—85°C, when "halo" is chloro. Polar, aprotic solvents which may be employed include dimethylsulfoxide, dimethylformamide, tetrahydrothiophene-1,1-dioxide, hexamethylphosphoric triamide, N-methylacetamide, ethylene carbonate, dimethoxy ethane and 1,4-dioxane. Non-nucleophilic, hydroxylated cosolvents described above may advantageously be employed at 1—10 parts per 100 parts of the polar, aprotic solvent. If the free phenols are employed, the reaction must be conducted in the presence of an alkali metal or alkaline earth metal base such as anhydrous hydroxides, carbonates, bicarbonates and the like; preferred alkali metals are potassium and sodium.

Synthesis of 5-chloro-2-nitrobenzoates (ie X″ is carboxy or carboxy esters) are described in Beilstein, IX, page 401 and may be illustrated as follows:

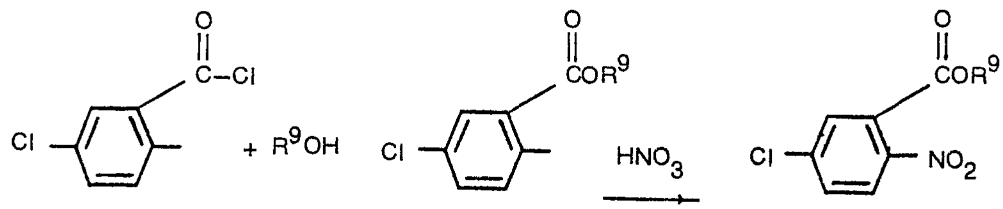


wherein $R^9$ is hydrogen or alkyl.

The synthesis of 5-fluoro-2-nitrobenzoic acid from 3-fluoro-benzoic acid has been described by J. H. Slothouwer, *Rec. trav. chim, 33,* 324—42, 1914.

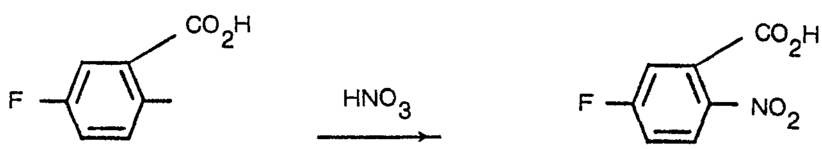


mp 134.5°C

The following preparations are included by way of guidance as to the use of the phenates of Formula I, and their corresponding free phenols, in the synthesis of the diphenyl ethers of Formula II; also included are preparations for various precursors usable in the synthesis of the diphenyl ethers of Formula II.

Preparation of halo 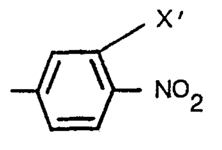

Preparation 1

*Methyl 5-Chloro-2-nitrobenzoate*

5-Chloro-2-nitrobenzoic acid (20.1 g, 0.10 mol), was dissolved in anhydrous methanol (100 ml) and anhydrous hydrogen chloride gas was bubbled into the reaction mixture for 4 hours at room temperature. The reaction mixture was then poured into water and the aqueous mixture triturated with hexane, the hexane layer decanted, dried (anhydrous $MgSO_4$) and the solvent removed under vacuum to afford 11.7 g of methyl 5-chloro-2-nitrobenzoate, mp 50—51°C.

Preparation 2

*Methyl 3-Fluorobenzoate*

To a solution of anhydrous hydrochloric acid (10.5 g) in anhydrous methanol (100 ml) was added *m*-fluorobenzoic acid (13.3 g, 0.095 mole). The reaction mixture was stirred at room temperature overnight. The solvent and anhydrous hydrochloric acid were then removed under vacuum and the residue slurried with hexane (100 ml). The hexane insoluble layer was decanted and the hexane solution washed with water (2 × 100 ml) before drying with anhydrous magnesium sulfate. The hexane filtrate was then concentrated under vacuum to afford 11.0 g (75% yield) of methyl 3-fluorobenzoate as a yellow liquid. Elemental anal. calcd. for $C_8H_7FO_2$: C, 62.35; H, 4.57; F, 12.33. Found: C, 62.52; H, 4.70; F, 11.89.

Preparation 3

*Methyl 3-Fluorobenzoate*

To a 1-liter flask fitted with a stirrer, addition funnel, condenser, and drying tube containing diethyl ether (200 ml) and methanol (anhydrous; 64 g, 2.0 moles) was added dropwise *m*-fluorobenzoyl chloride (317 g, 2.0 moles) at room temperature over a 3 hour period. Additional methanol (10 ml) was added and the reaction mixture was stirred at room temperature overnight. The reaction mixture was then filtered through a bed of silicic acid and the solvent removed under vacuum to afford 285 grams of methyl 3-fluorobenzoate (92.5% yield). This was identical to the product prepared in Preparation 2.

By following substantially the procedure of Preparation 3 and by substituting for the methanol recited therein an equimolar quantity of another alcohol there are obtained other desired esters. The following Table II taken together with the flow diagram illustrates these preparations:

TABLE II

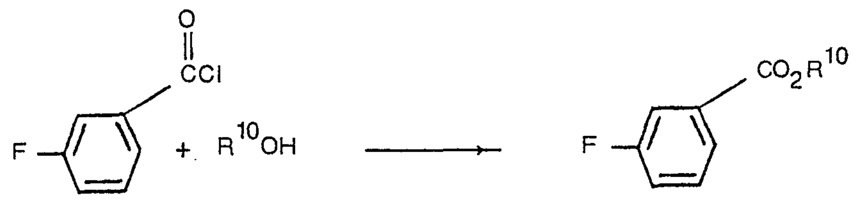

| Preparation No. | $R^{10}$ | Molecular Formula | Elemental Analysis |
|---|---|---|---|
| 4 | $-C_2H_5$ | $C_9H_9FO_2$ | — |
| 5 | $-CH_2CH_2N(CH_3)_2$ | $C_{11}H_{14}FNO_2$ | Calcd: C, 62.50;<br>H, 6.70;<br>F, 9.00;<br>Found: C, 62.25;<br>H, 6.76;<br>F, 8.48; |
| 6 | $-C_8H_{17}$ | $C_{15}H_{21}FO_2$ | Calcd: C, 71.40;<br>H, 8.38;<br>F, 7.52;<br>Found: C, 71.45;<br>H, 8.57;<br>F, 7.37; |
| 7 | $CH_3$<br>$-CHCH_3$ | $C_{10}H_{10}FO_2$ | Calcd: C, 66.25;<br>H, 5.57;<br>F, 10.50;<br>Found: C, 65.88;<br>H, 5.79;<br>F, 10.13; |
| 8 | $-CH_2CH_2CH_2CH_3$ | $C_{11}H_{13}FO_2$ | — |
| 9 | $-CH_2CH_2OH$ | $C_9H_9FO_3$ | — |
| 10 | $-CH_2CH_2CH_3$ | $C_{10}H_{11}FO_2$ | — |
| 11 | $-CH_2CH_2OCH_3$ | $C_{10}H_{11}FO_3$ | — |

Preparation 12

*N,N-Diethyl-3-fluorobenzamide*

To a 50 ml flask fitted with a stirrer, condenser, drying tube and an addition funnel was added *m*-fluorobenzoyl chloride (15.9 g, 0.10 mole) and benzene (100 ml). Then a solution of diethylamine (14.6 g, 0.20 mole) in benzene (100 ml) was added dropwise over 1 hour and the reaction mixture was stirred at room temperature overnight. Water (150 ml) was then added rapidly and after trituration for a few minutes, the benzene layer was decanted and washed repeatedly with water (5 x 150 ml), dried over anhydrous $Na_2SO_4$ and the solvent removed under vacuum to afford 18 grams of N,N-diethyl-3-fluorobenzamide as a yellow oil. Elemental analysis calculated for $C_{11}H_{14}FNO$: C, 67.70; H, 7.22; F, 9.72; N, 7.16. Found: C, 66.40; H, 7.08; F, 9.65; N, 6.92.

Preparation 13

*3-Fluorobenzamide*

Into a stirred solution of *m*-fluorobenzoyl chloride (15.9 g, 0.10 mole) in benzene (200 ml) at 5°C was bubbled anhydrous ammonia gas for 30 minutes. A white solid precipitated and the reaction mixture was stirred at room temperature overnight. The white solid which resulted was removed by filtration and then triturated with water. The solid was dried to afford 10 g of *m*-fluorobenzamide, mp 130°—33°C. Elemental analysis calculated for $C_7H_6FNO$: C, 60.40; H, 4.34; F, 13.66; N, 10.08. Found: C, 59.97; H, 4.43; F, 13.86; N, 10.39.

*5-Fluoro-2-Nitrobenzoate Esters and Amides*

By a modification of the method of Slothouwer (*loc. cit.*) in which is described the preparation of 5-fluoro-2-nitrobenzoic by nitration of 3-fluorobenzoic acid, under standard nitration conditions, the 3-fluorobenzoate esters and benzamides were nitrated as follows:

Preparation 14

*Methyl 5-Fluoro-2-nitrobenzoate*

To a 500 ml, 4-necked flask fitted with a stirrer, thermometer, addition funnel and reflux condenser was charged sulfuric acid (50 ml) and methyl *m*-fluorobenzoate (8.5 g, 0.055 mole). The reaction mixture was then cooled to 5°C and a solution of potassium nitrate (reagent grade 5.57 g, 0.055 mole) in concentrated sulfuric acid (50 ml) was added dropwise over a 30 minute period. Ten minutes after completion of such addition, the reaction mixture was poured onto 500 g of cracked ice and the product was extracted into benzene (3 × 100 ml). The combined benzene extracts were dried over anhydrous magnesium sulfate and the filtrate reduced *in vacuo* to give 10.6 g (96%) of product, which is a yellow liquid. *Anal.* Calcd. for $C_8H_6FNO_4$: C, 48.20; H, 3.04; F, 9.54; N, 7.02. Found: C, 50.15; H, 3.34; F, 9.17; N, 6.77.

By following substantially the procedure of Preparation 14 and by substituting an appropriate ester or amide of 3-fluorobenzoic acid for the ester recited therein the products depicted in Table III are prepared.

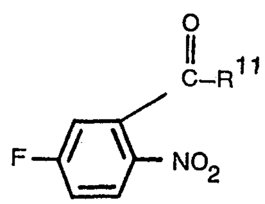

TABLE III

| Preparation No. | $R^{11}$ | Molecular Formula | Elemental Anal. |
|---|---|---|---|
| 15 | —OH | $C_7H_4NO_4$ | Calcd: C, 45.40;<br>H, 2.18;<br>F, 10.25;<br>Found: C, 45.02;<br>H, 2.13;<br>F, 9.84; |
| 16 | $—OC_2H_5$ | $C_9H_8FNO_4$ | — |
| 17 | $—OC_8H_{17}$ | $C_{15}H_{20}FO_4$ | Calcd: C, 60.60;<br>H, 6.78;<br>F, 6.40;<br>Found: C, 63.10;<br>H, 7.08;<br>F, 6.50; |
| 18 | $—N(C_2H_5)_2$ | $C_{11}H_{13}FN_2O_3$ | Calcd: C, 55.00;<br>H, 5.45;<br>F, 7.90;<br>Found: C, 54.89;<br>H, 5.46;<br>F, 7.82; |
| 19 | $CH_3$<br>\|<br>$—OCHCH_3$ | $C_{10}H_9FNO_4$ | — |
| 20 | $—NH_2$ | $C_7H_5FN_2O_3$ | — |
| 21 | $—O(CH_2)_3CH_3$ | $C_{11}H_{12}NO_4$ | — |
| 22 | $—OCH_2CH_2CH_3$ | $C_{10}H_{10}FNO_4$ | Calcd: C, 52.86;<br>H, 4.43;<br>F, 6.16;<br>Found: C, 53.34;<br>H, 4.40;<br>F, 5.97; |
| 23 | $—OCH_2CH_2OCH_3$ | $C_{10}H_{10}FNO_5$ | Calcd: C, 49.58;<br>H, 4.14;<br>F, 5.75;<br>Found: C, 49.57;<br>H, 4.00;<br>F, 5.85; |
| 24 | $—OCH_2CH_2OH$ | $C_9H_8FNO_5$ | Calcd: C, 47.37;<br>H, 3.53;<br>F, 7.91;<br>Found: C, 47.53;<br>H, 3.74;<br>F, 7.73. |

Preparation 25

*Ethyl 2-(5-Fluoro-2-nitrophenoxy)propionate*

2,4-Difluoronitrobenzene (15.9 g; 0.10 mole), ethyl lactate (11.8 g; 0.10 mole) and anhydrous potassium carbonate (13.8 g; 0.10 mole) were combined in dioxane (200 ml) and the temperature was brought to 95°C over a 1.5 hour period and maintained at 95°C for 3 hours. The reaction mixture was cooled to room temperature, the inorganic solids removed by filtration and the solvent removed under vacuum to afford a dark coloured oil, from which low boiling fractions were removed by distillation (bp., 79°C/0.5 mm). The pot residue was then cooled to afford 15.9 g of crude product, which was recrystallized from ligroin/acetone to afford ethyl 2-(5-fluoro-2-nitrophenoxy)propionate as yellow needles, m.p. 64—65°C. Elemental anal. Calcd. for $C_{11}H_{12}FNO_5$: C, 51.4; H, 4.70; F, 7.38; N, 5.45. Found: C, 51.35; H, 4.74; F, 7.60; N, 5.44.

Preparation 26

*2-Ethoxy-4-fluoronitrobenzene*

To a solution of anhydrous sodium ethoxide (0.55 mole) (prepared by dissolving sodium metal (12.6 g, 0.55 mole) in anhydrous ethanol (300 ml)) at 0°C was added 2,4-difluoronitrobenzene (79.5 g, 0.50 mole) dropwise over an 8 hour period. Upon completion of addition the reaction mixture was allowed to warm to room temperature and stirred overnight. The resulting solid was removed by filtration, and triturated with toluene and water. The toluene extract was combined with the original filtrate, dried over anhydrous sodium sulfate and the solvent removed under vacuum to afford 218 g of a yellow oil which was distilled at 0.3 mm, b.p. 95—98°C to afford substantially pure 2-ethoxy-4-fluoronitrobenzene.

Preparation of 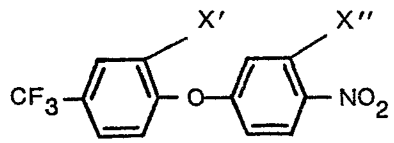

Preparation 27

*Methyl 5(2-chloro-4-trifluoromethylphenoxy)2-nitrobenzoate*

To a 5-litre, 4-necked flask fitted with a stirrer, thermometer, addition funnel, reflux condenser and drying tube was added dimethyl sulfoxide (200 ml), 2-chloro-4-trifluoromethylphenol (715 g, 3.64 mole), and anhydrous potassium carbonate (520 g, 3.72 moles). The reaction mixture was stirred at room temperature overnight under a nitrogen atmosphere. Then methyl 5-fluoro-2-nitrobenzoate (724 g, 3.64 moles) was added over a 1 hour period (slight exotherm to 31°C). After stirring overnight at room temperature, the inorganic solids were removed by filtration and 1700 ml of dimethyl sulfoxide removed under vacuum (1 mm, pot temp. of 85°C, vapour temp. of —40°C). The residue was diluted with a large volume of carbon tetrachloride and water washed. The organic layer was dried over anhydrous magnesium sulfate and the solvent removed under vacuum to afford 1142 g of methyl 5-(2-chloro-4-trifluoromethylphenoxy)-2-nitrobenzoate (84% yield). *Anal.* Calcd. for $C_{15}H_9ClF_3NO_5$: C, 47.90; H, 2.42; Cl, 9.45; F, 15.20; N, 3.73. Found: C, 47.83; H, 2.47; Cl, 9.15; F, 14.84; N, 3.69.

By following the procedure of Preparation 27 and by varying the temperature, time and solvent a repeat was made of the methyl ester. Also prepared was the ethyl ester and several other esters. The following Table IVA illustrates these preparations and Table IVB gives representative elemental analyses.

TABLE IVA

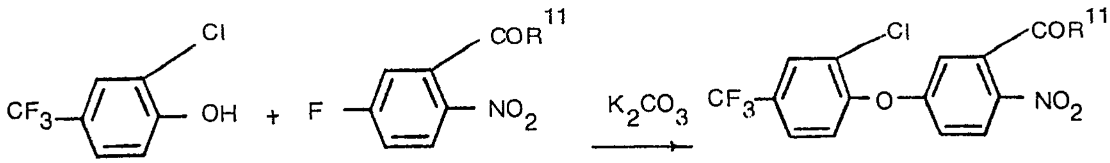


| Preparation No. | $R^{11}$ | Time (hours) | Temp (°C) | Solvent | Yield (%) |
|---|---|---|---|---|---|
| 28 | $—OH$ | 8 | 70—100 | DMSO | 1 |
| 29 | $—OCH_3$ | 1 | 70 | DMSO | 30 |
| 30 | $—OCH_3$ | 8 | reflux | dioxane | 10 |
| 31 | $—OCH_3$ | 48 | 25 | DMSO | 87 |
| 32 | $—OCH_3$ | 5 | 25 | DMSO | 84 |
| 33 | $—OCH_3$ | 8 | 25—48 | DMSO | 77 |
| 34 | $—OC_2H_5$ | 24 | 25 | DMSO | 68 |
| 35 | $—OC_2H_5$ | 72 | 25—70 | DMSO | 86 |
| 36 | $—OC_2H_5$ | 8 | 40 | DMSO | 90 |
| 37 | $—OC_3H_7$*n* | 24 | 25 | DMSO | 88 |
| 38 | $—OC_3H_7$*iso* | 96 | 25 | DMSO | 55 |
| 39 | $—OC_4H_9$*n* | 72 | 25 | DMSO | 81 |
| 40 | $—OC_8H_{17}—$*n* | 72 | 25 | DMSO | 48 |
| 41 | $—OCH_2CH_2OH$ | 24 | 25 | DMSO | 59 |
| 42 | $—OCH_2CH_2OCH_3$ | 24 | 25 | DMSO | 90 |
| 43 | $—OCH_2CH_2N(CH_3)_2$ | 24 | 25—45 | DMSO | 65 |

TABLE IVB

| Preparation No. | MOLECULAR FORMULA | | ELEMENTAL ANALYSIS C | H | Cl | F | N |
|---|---|---|---|---|---|---|---|
| 34 | $C_{16}H_{11}ClF_3NO_5$ | THEORY | 49.31 | 2.84 | 9.09 | 14.63 | 3.59 |
| | | FOUND | 49.70 | 2.96 | 8.85 | 14.15 | 3.30 |
| 37 | $C_{17}H_{13}ClF_3NO_5$ | THEORY | 50.57 | 3.24 | 8.78 | 14.17 | 3.47 |
| | | FOUND | 50.85 | 3.50 | 8.70 | 13.70 | 3.35 |
| 38 | $C_{17}H_{13}ClF_3NO_5$ | THEORY | 50.57 | 3.24 | 8.78 | 14.17 | 3.47 |
| | | FOUND | 50.56 | 3.26 | 8.96 | 14.41 | 3.41 |
| 39 | $C_{18}H_{15}ClF_3NO_5$ | THEORY | 51.75 | 3.61 | 8.48 | 13.64 | 3.35 |
| | | FOUND | 51.87 | 3.86 | 8.47 | 13.52 | 3.13 |
| 40 | $C_{22}H_{23}ClF_3NO_5$ | THEORY | 55.75 | 4.88 | 7.48 | 12.03 | 2.97 |
| | | FOUND | 56.14 | 5.10 | 7.58 | 11.48 | 2.76 |
| 41 | $C_{16}H_{11}ClF_3NO_6$ | THEORY | 47.36 | 2.73 | 8.73 | 14.04 | 3.05 |
| | | FOUND | 47.10 | 2.72 | 8.58 | 16.99 | 3.29 |
| 42 | $C_{16}H_{11}ClF_3NO_6$ | THEORY | 48.64 | 3.12 | 8.44 | 13.57 | 3.33 |
| | | FOUND | 48.40 | 3.23 | 8.68 | 13.18 | 3.17 |
| 43 | $C_{18}H_{16}ClF_3N_2O_5$ | THEORY | 49.95 | 3.73 | 8.19 | 13.17 | 6.47 |
| | | FOUND | 48.86 | 3.69 | 8.14 | 13.59 | 6.16 |

Preparation 44

*5-(2-Chloro-4-trifluoromethylphenoxy)-2-nitrobenzamide*

To a 100 ml flask fitted with a magnetic stirrer and drying tube was added dimethyl sulfoxide (50 ml), anhydrous potassium carbonate (3.6 g, 0.026 mole) and 2-chloro-4-trifluoromethylphenol (4.8 g, 0.024 mole). This mixture was stirred at room temperature overnight. 5-Fluoro-2-nitrobenzamide (4.5 g, 0.024 mole) was added rapidly and the reaction mixture stirred at room temperature for 24 hours. The reaction mixture was then poured into water (300 ml) and triturated with carbon tetrachloride (50 ml). The water and carbon tetrachloride insoluble solid was collected by filtration and dried under vacuum to afford 1.2 g of 5-(2-chloro-4-trifluoromethylphenoxy)-2-nitrobenzamide, mp 139—43°C. *Anal.* Calcd. for $C_{14}H_8ClF_3N_2O_4$: C, 46.62; H, 2.23; Cl, 9.83; N, 7.76. Found: C, 45.77; H, 2.30; Cl, 10.62; N, 7.91.

Preparation 45

*N,N-Diethyl-5-(2-Chloro-4-trifluoromethylphenoxy)-2-nitrobenzamide*

To a 100 ml flask fitted with a magnetic stirrer and drying tube was added dimethyl sulfoxide (30 ml), anhydrous potassium carbonate (7.55 g, 0.054 mole) and 2-chloro-4-trifluoromethylphenol (10.3 g, 0.052 mole). The mixture was stirred at room temperature overnight. A solution of N,N-diethyl-5-fluoro-2-nitrobenzamide (12.5 g, 0.052 mole) in dimethyl sulfoxide (20 ml) was then added rapidly and the reaction mixture stirred at room temperature for 60 hours. The reaction mixture was then poured into water (500 ml) and the product extracted into carbon tetrachloride (4 × 100 ml), the combined extract being dried over anhydrous magnesium sulfate and the solvent removed under vacuum to afford 15.3 g of 5-(2-chloro-4-trifluoromethylphenoxy)-2-nitrobenzamide (75% yield). Recrystallization from benzene/hexane afforded substantially pure product mp. 75—79°C. Elemental analysis, Calcd. for $C_{18}H_{16}ClF_3N_2O_4$: C, 51.80; H, 3.88; Cl, 8.55; F, 13.68; N, 6.73. Found: C, 52.03; H, 3.90; Cl, 8.54; F, 13.59; N, 6.83.

Preparation 46

*Methyl 5-(2-Chloro-4-trifluoromethylphenoxy)-2-nitrobenzoate*

To a flask fitted with a drying tube was charged DMSO (10 ml), 2-chloro-4-trifluoromethylphenol (3.93 g, 0.02 mole) and anhydrous potassium carbonate (2.76 g, 0.02 mole). Then methyl 5-chloro-2-nitrobenzoate (4.3 g, 0.02 mole) was added and the reaction mixture warmed to 50°C for 3 hours. (Gas-liquid chromatography analysis suggested a low conversion to desired product). The temperature was raised to 60°C for 16 hours and then to 70°C for 16 hours. Additional potassium carbonate (1.38 g, 0.01 mole) was added and heating at 70°C continued for an additional 5 days, the reaction mixture

then being cooled to room temperature, poured into a benzene/hexane cosolvent and extracted twice with water. The organic layer was dried over anhydrous sodium sulfate, filtered and the solvent removed under vacuum (110°C, 0.1 mm) to afford 2.0 g, (27% yield) of methyl 5-(2-chloro-4-trifluoromethylphenoxy)-2-nitrobenzoate which was determined by spectral data to be identical with the product of Preparation 27.

Preparation 27

*Methyl 5-(2-Chloro-4-trifluoromethylphenoxy)-2-nitrobenzoate*

Following the procedure of Preparation 46, tetrahydrothiophene-1,1-dioxide was substituted for DMSO and the reaction temperature was maintained at 115°C for 24 hours to afford 2 g of methyl 5-(2-chloro-4-trifluoromethylphenoxy)-2-nitrobenzoate.

Preparation 48

*Methyl 5-(2-Chloro-4-trifluoromethylphenoxy)-2-nitrobenzoate*

The potassium salt of 2-chloro-4-trifluoromethylphenol was prepared by adding, at 0—5°C, one equivalent of 2-chloro-4-trifluoromethylphenol to a solution of 1 equivalent of potassium hydroxide dissolved in methanol. The solvent was removed under vacuum and tetrahydrothiophene-1,1-dioxide was added to dissolve the anhydrous potassium salt (II). This solution was then added dropwise to 1 equivalent of methyl 5-chloro-2-nitrobenzoate dissolved in tetrahydrothioxene-1,1-dioxide preheated to 105°C. Upon completion of addition the temperature was raised to 125°C for 3 hours, then cooled to room temperature, poured into water and extracted into carbon tetrachloride. GLC analysis indicated that the yield was comparable to that of Preparation 47.

By following the general procedures of Preparations 27, 44, 45 or 46 and selecting the appropriate 4-trifluoromethylphenol and halonitrobenzene there can also be prepared the compounds illustrated in Table V:

TABLE V

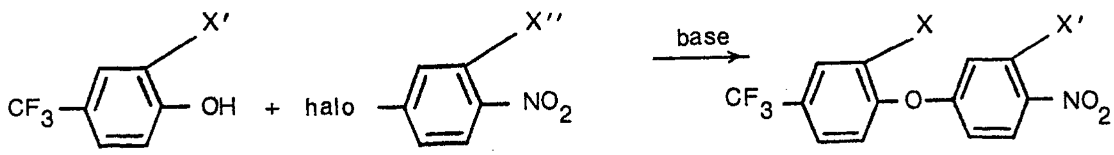


| Preparation No. | X' | X'' | m.p. (°C) | bp (°C)/torr |
|---|---|---|---|---|
| 49 | Cl | $-OC_2H_5$ | 83—84 | — |
| 50 | Cl | $-O\overset{CH_3}{\overset{\vert}{C}}H-\overset{O}{\overset{\Vert}{C}}OC_2H_5$ | 71—72.5 | — |
| 51 | Cl | $-\overset{O}{\overset{\Vert}{C}}OCH_2CH=CH_2$ | oil | 180/03 |
| 52 | Cl | $-\overset{O}{\overset{\Vert}{C}}NHCH_3$ | 118—20°C | — |
| 53 | Cl | $-\overset{O}{\overset{\Vert}{C}}N(CH_3)_2$ | oil | 180/0.3 |
| 54 | Cl | $-\overset{O}{\overset{\Vert}{C}}OH$ | 156—60 | — |
| 55 | Cl | $CH_3$ | — | 135/0.08 |
| 56 | Cl | $-\overset{O}{\overset{\Vert}{C}}OC_4H_9-t$ | oil | 180/0.3 |

The product of Preparation 54 can also be prepared by acid or base catalyzed ester hydrolysis of the products of Preparations 27 or 34—43, one illustration of which is described in Preparation 57 following.

Preparation 57

*5-(2-Chloro-4-trifluoromethylphenoxy)-2-nitrobenzoic acid*

Ethyl 5-(2-chloro-4-trifluoromethylphenoxy)-2-nitrobenzoate (Preparation 34, 20 g, 0.058 mole) was dissolved in ethanol (20 g) and a solution of 50% aqueous sodium hydroxide (9.6 g, 4 g, 0.120 mole) was added dropwise beginning at 25°C. The rate of addition was adjusted so as to control the exothermic reaction to a temperature of less than or equal to 52°C. By this method the sodium salt of 5-(2-chloro-4-trifluoromethylphenoxy)-2-nitrobenzoic acid was generated *in situ*. Treatment with 6 M HCl to pH 1 in water (100 ml) afforded the free acid as a heavy oil which was extracted into ethylene chloride. The ethylene chloride layer was decanted and concentrated under vacuum to afford 17.6 g of a thick brown syrupy material which crystallized upon further trituration with ethylene dichloride as an off-white powder, mp 156—60°C which was identified by spectral means to be 5-(2-chloro-4-trifluoromethylphenoxy)-2-nitrobenzoic acid.

By standard chemical methods 5-(2-chloro-4-trifluoromethylphenoxy)-2-nitrobenzoic acid can be converted to agronomically acceptable alkali and alkaline earth metal salts by reaction with one equivalent of an appropriate metal hydroxide or to agronomically acceptable amine salts by combining the acid with one equivalent of the appropriate amine. Solutions of the acid salts or free salts can be obtained depending upon reaction conditions. One method of isolating the sodium salt is described in Preparation 58.

Preparation 58

*Sodium 5-(2-Chloro-4-trifluoromethylphenoxy)-2-nitrobenzoate*

5-(2-chloro-4-trifluoromethylphenoxy)-2-nitrobenzoic acid (3.0 g, 0.0083 mole) was dissolved in *iso*-propyl alcohol (20 ml) and a solution of 50% aqueous sodium hydroxide (0.644 g, 0.0083 mole) was added dropwise and the reaction mixture stirred for 1 hour before removing the solvent and water under vacuum at 0.2 mm overnight to afford 2.8 g of sodium 5-(2-chloro-4-trifluoromethylphenoxy)-2-nitrobenzoate.

Preparation 59

*Methyl 5-(4-Chloro-2-trifluoromethylphenoxy)-2-nitrobenzoate*

To a flask was added DMSO (10 ml) 4-chloro-2-trifluoromethylphenol (3.0 g, 0.015 mole), anhydrous potassium carbonate (2.1 g, 0.015 mole) and methyl 5-fluoro-2-nitrobenzoate (2.99 g, 0.015 mole). The reaction mixture was stirred at room temperature for 7 hours, poured in water (150 ml) and then triturated with $CCl_4$ (50 ml then 35 ml) and the organic layer decanted and removed under vacuum to afford 5.3 g of product as a yellow oil. Elemental anal. Calcd. for $C_{15}H_9ClF_3NO_5$: C, 47.90; H, 2.42; Cl, 9.68; F, 15.20; N, 3.73. Found: C, 47.70; H, 2.49; Cl, 9.61; F, 14.89; N, 3.31.

**Claims**

1. A process for preparing a substituted phenate or the corresponding phenol, said phenate being of the formula:

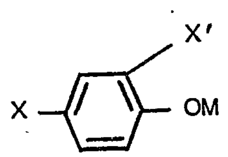

(I)

wherein X is halo or trifluoromethyl with the proviso that, when X is halo, X′ is trifluoromethyl,

X′ is hydrogen, halo, trifluoromethyl or ($C_1$—$C_4$) alkyl, and

M is the cation of an alkali metal or alkaline earth metal,

the process comprising reacting a compound of the formula:

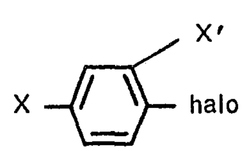

(wherein X and X′ are as above defined) with an alkali metal or alkaline earth metal hydroxide base in a solvent system which is substantially inert to said base characterized in that the solvent system

employed comprises a polar, aprotic solvent with a dielectric constant in the range of from 30 to 70 and a non-nucleophilic hydroxy-containing solvent which increases the solubility of said base in the solvent system whereby a phenate is obtained and, if a phenol is the desired product, reacting the phenate with an acid to form the phenol.

2. A process as claimed in Claim 1, wherein X is trifluoromethyl and X' is chloro.

3. A process as claimed in Claim 1 or 2, wherein the non-nucleophilic hydroxy containing solvent is selected from:

a)

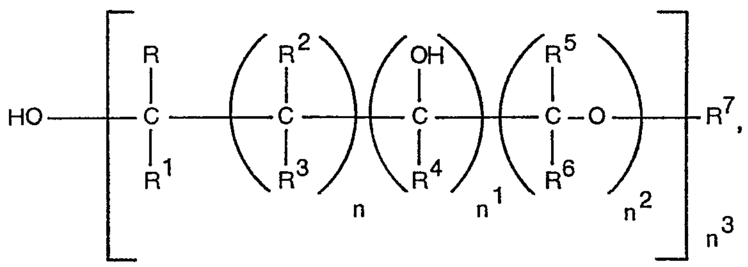

b)

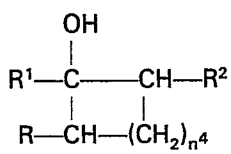

c) $C(CH_2OH)_4$ or

d)

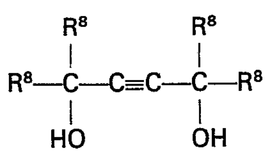

wherein R, $R^1$, $R^2$, $R^3$, $R^4$, $R^5$, $R^6$ and $R^7$ are the same or different groups selected from hydrogen; straight or branched $(C_1$—$C_4)$alkyl; unsubstituted or substituted phenyl wherein the phenyl substituent(s) is or are selected from $(C_1$—$C_4)$alkyl, halo, $(C_1$—$C_4)$alkoxy and benzyl; $R^8$ is $(C_1$—$C_4)$alkyl; n and $n^2$ are 0 or 1; $n^1$ is 0 to 2; $n^3$ is an integer of from 1 to 10 and $n^4$ is 2 to 4; provided that (a) $n^1$ is 2 only when n and $n^2$ are both 0, otherwise the sum of $n^1$ and $n^2$ must not be greater than 1, (b) (when $n^3$ is greater than 2) the sum of n and $n^2$ must not be greater than 1 and $n^1$ must be 0, and (c) (when $R^7$ is hydrogen) $n^1$ must be zero, the sum of n and $n^2$ must be 1 and at least 3 of R, $R^1$ plus either the pair $R^2$ and $R^3$ or the pair $R^5$ and $R^6$ must not be hydrogen.

4. A process as claimed in any one of Claims 1 to 3, wherein the polar aprotic solvent is dimethyl sulfoxide, tetrahydrothiophene-1,1-dioxide or hexamethylphosphoric triamide.

5. A process as claimed in any one of Claims 1 to 4, wherein the solvents are employed in a volume ratio of from 1 part to 30 parts of the non-nucleophilic hydroxy solvent to 100 parts of the polar, aprotic solvent.

6. A process as claimed in any one of Claims 1 to 5, wherein there is employed, as the base, sodium or potassium hydroxide.

7. A process as claimed in any preceding claim, wherein the reaction to form the phenate is carried out at a temperature from 50° to 100°C.

**Patentansprüche**

1. Verfahren zur Herstellung eines substituierten Phenats der entsprechenden Formel, wobei das Phenat die Formel

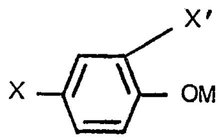

(I)

besitzt, worin X Halogen oder Trifluormethyl ist, unter der Voraussetzung, daß dann, wenn X für Halogen steht, X′ Trifluormethyl bedeutet, X′ Wasserstoff, Halogen, Trifluormethyl oder $(C_1$—$C_4)$Alkyl ist und M das Kation eines Alkalimetalls oder eines Erdalkalimetalls bedeutet, dadurch gekennzeichnet, daß eine Verbindung der Formel

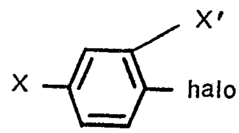

worin X und X′ die vorstehend angegebenen Bedeutungen besitzen, mit einer Alkali- oder Erdalkalimetallhydroxidbase in einem Lösungsmittelsystem umgesetzt werden, das im wesentlichen gegenüber der Base inert ist, dadurch gekennzeichnet, daß das verwendete Lösungsmittelsystem aus einem polaren aprotischen Lösungsmittel mit einer Dielektrizitätskonstante im Bereich von 30 bis 70 und einem nichtnukleophilen hydroxyenthaltenden Lösungsmittel, welches die Löslichkeit der Base in dem Lösungsmittelsystem erhöht, besteht, wobei ein Phenat erhalten ist und, falls ein Phenol das gesuchte Produkt ist, das Phenat mit einer Säure unter Bildung des Phenols zur Umsetzung gebracht wird.

2. Verfahren nach Anspruch 1, dadurch gekennzeichnet, daß X für Trifluormethyl steht und X′ Chlor bedeutet.

3. Verfahren nach Anspruch 1 oder 2, dadurch gekennzeichnet, daß das eingesetzte nichtnukleophile hydroxyenthaltende Lösungsmittel aus

a) 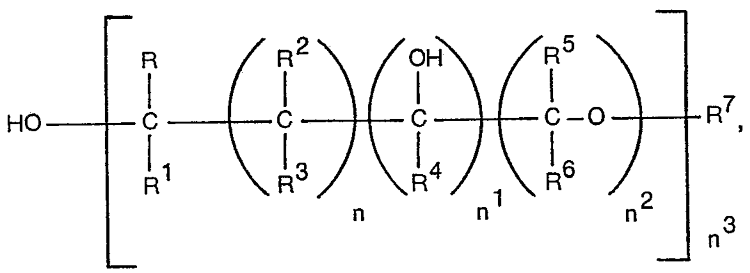

b) 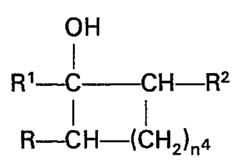

c) $C(CH_2OH)_4$ oder

d) 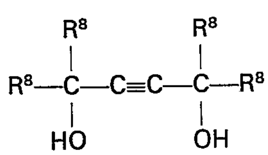

besteht, wobei R, $R^1$, $R^2$, $R^3$, $R^4$, $R^5$, $R^6$ und $R^7$ gleich oder verschiedene Gruppen sind, ausgewählt aus Wasserstoff, geradkettigem oder verzweigtem ($C_1$—$C_4$)Alkyl, nichtsubstituiertem oder substituiertem Phenyl, wobei der Phenylsubstituent bzw. die Phenylsubstituenten aus ($C_1$—$C_4$)Alkyl, Halogen, ($C_1$—$C_4$)Alkoxy oder Benzyl besteht (bestehen), $R^8$ ($C_1$—$C_4$)Alkyl ist, n und $n^2$ 0 oder 1 sind, $n^1$ 0 bis 2 bedeutet, $n^3$ eine ganze Zahl von 1 bis 10 ist und $n^4$ 2 bis 4 ist, unter der Voraussetzung, daß (a) $n^1$ nur dann 2 bedeutet, wenn n und $n^2$ jeweils 0 sind, und ansonsten die Summe von $n^1$ und $n^2$ nicht größer als 1 sein darf, (b) dann, wenn $n^3$ größer als 2 ist, die Summe von n und $n^2$ nicht größer als 1 sein darf und $n^1$ 0 sein muß, und (c) dann, wenn $R^7$ für Wasserstoff steht, $n^1$ 0 sein muß, wobei die Summe von n und $n^2$ 1 sein muß, und wenigstens drei der Substituenten R, $R^1$ sowie einer der Substituenten des Paares $R^2$ und $R^3$ oder des Paares $R^5$ und $R^6$ kein Wasserstoff sein darf.

4. Verfahren nach einem der Ansprüche 1 bis 3, dadurch gekennzeichnet, daß das verwendete polare aprotische Lösungsmittel aus Dimethylsulfoxid, Tetrahydrothiophen-1,1-dioxid oder Hexamethylphosphorsäuretriamid besteht.

5. Verfahren nach einem der Ansprüche 1 bis 4, dadurch gekennzeichnet, daß die Lösungsmittel in einem Volumenverhältnis von 1 Teil bis 30 Teil des nichtnukleophilen Hydroxylösungsmittels zu 100 Teilen des polaren aprotischen Lösungsmittels verwendet werden.

6. Verfahren nach einem der Ansprüche 1 bis 5, dadurch gekennzeichnet, daß als Base Natrium- oder Kaliumhydroxid verwendet wird.

7. Verfahren nach einem der vorhergehenden Ansprüche, dadurch gekennzeichnet, daß die Reaktion zur Bildung des Phenats bei einer Temperatur von 50 bis 100°C durchgeführt wird.

**Revendications**

1. Procédé pour préparer un phénate substitué ou le phénol correspondant, ledit phénate répondant à la formule:

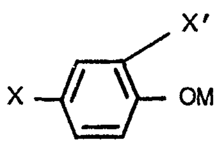

(I)

où:

X est un halogéno ou un trifluorométhyle sous réserve que lorsque X est un halogéno, X′ est un trifluorométhyle,

X′ est un hydrogène, un halogéno, un trifluorométhyle ou un alcoyle en $C_1$—$C_4$, et

M est le cation d'un métal alcalin ou d'un métal alcalinoterreux,

le procédé comprenant la réaction d'un composé de formule:

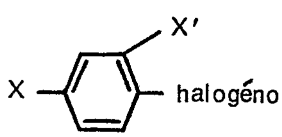

(où X et X′ sont comme défini ci-dessus) avec une base constituée d'un hydroxyde de métal alcalin ou de métal alcalinoterreux, dans un système solvant qui est pratiquement inerte vis-à-vis de ladite base, caractérisé en ce que le système solvant employé comprend un solvant aprotique polaire ayant une constante diélectrique dans la gamme de 30 à 70 et un solvant hydroxylé non nucléophile qui accroît la solubilité de ladite base dans le système solvant pour obtenir un phénate et, si le produit désiré est un phénol, la réaction du phénate avec un acide pour former le phénol.

2. Procédé comme revendique dans la revendication 1 où X est un trifluorométhyle et X′ est un chloro.

3. Procédé comme revendiqué dans la revendication 1 ou 2, où le solvant hydroxylé non nucléophile est choisi parmi:

a)

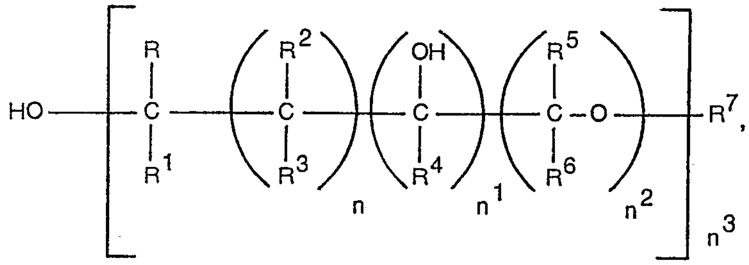

b)

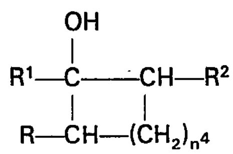

c)

$C(CH_2OH)_4$ ou

d)

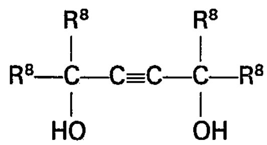

où R, $R^1$, $R^2$, $R^3$, $R^4$, $R^5$, $R^6$ et $R^7$ sont des radicaux semblables ou différents choisis parmi un hydrogène; un alcoyle droit ou ramifié en $C_1$—$C_4$; un phényle non substitué ou substitué dont le(s) substituant(s) est(sont) choisi(s) parmi un alcoyle en $C_1$—$C_4$, un halogéno, un alcoxy en $C_1$—$C_4$ et un benzyle; $R^8$ est un alcoyle en $C_1$—$C_4$; n et $n^2$ sont 0 ou 1; $n^1$ est 0 à 2; $n^3$ est un nombre entier de 1 à 10 et $n^4$ est 2 à 4; sous réserve que (a) $n^1$ est 2 uniquement lorsque n et $n^2$ sont tous deux 0, autrement la somme de $n^1$ et $n^2$ ne doit pas dépasser 1, (b) (lorsque $n^3$ est supérieur à 2), la somme de n et $n^2$ ne doit pas être supérieure à 1 et $n^1$ doit être 0 et (c) (lorsque $R^7$ est un hydrogène), $n^1$ doit être 0, la somme de n et $n^2$ doit être 1 et au moins trois de R, $R^1$ plus soit la paire $R^2$ et $R^3$ soit la paire $R^5$ et $R^6$ ne doit pas être un hydrogène.

4. Procédé comme revendiqué dans l'une quelconque des revendications 1 à 3 où le solvant aprotique polaire est le diméthylsulfoxyde, le tétrahydrothiophène-1,1-dioxyde ou l'hexamèthylphosphorotriamide.

5. Procédé comme revendiqué dans l'une quelconque des revendications 1 à 4, où les solvants sont employés dans un rapport en volume de 1 partie à 30 parties du solvant hydroxylé non nucléophile à 100 parties du solvant aprotique polaire.

6. Procédé comme revendiqué dans l'une quelconque des revendications 1 à 5, où on emploie comme base l'hydroxyde de sodium ou de potassium.

7. Procédé comme revendiqué dans l'une quelconque des revendications précédentes, où la réaction pour former le phénate est effectuée à une température de 50 à 100°C.